Europäisches Patentamt

**European Patent Office**    (11) Publication number: **0 084 583**

Office européen des brevets                                    **A1**

(12) **EUROPEAN PATENT APPLICATION**

(21) Application number: **82100532.9**    (51) Int. Cl.³: **A 61 J 1/00**

(22) Date of filing: **27.01.82**

(43) Date of publication of application: **03.08.83**
Bulletin 83/31

(84) Designated Contracting States: **BE DE FR GB IT NL SE**

(71) Applicant: **Becton, Dickinson and Company, Mack Centre Drive, Paramus New Jersey 07652 (US)**

(72) Inventor: **Brown, Harold, 440 Viola Road, Spring Valley New York (US)**
Inventor: **Stern, Charles, 2250 East 4th Street, Brooklyn New York (US)**
Inventor: **Siuta, Ted, 348 New York Avenue, Elizabeth New Jersey (US)**

(74) Representative: **Selting, Günther, Dipl.-Ing. et al, Deichmannhaus am Hauptbahnhof, D-5000 Köln 1 (DE)**

(54) **Magnification guide for syringes.**

(57) A magnification guide (10) for use in filling or emptying a liquid transfer instrument (30), such as a syringe, in conjuction with a liquid container (25) includes a transparent magnifying member (12). This member (12) includes a recess (19) for retaining a liquid container (25) thereon during use and a guide channel (16) for guiding the instrument (30) into liquid communication with the container (25) during use. The magnifying member (12) is adapted to magnify the surface of the instrument (30) when positioned in the guide channel (16).

425-9-1

1

## MAGNIFICATION GUIDE FOR SYRINGES

### BACKGROUND OF THE INVENTION

1. <u>Field of the Invention</u>. The present invention relates to a magnification guide for use in filling or emptying a liquid transfer instrument in conjunction with a liquid container, and more particularly, concerns such a guide useful in helping patients manipulate a syringe in extracting liquid medication from a liquid container while improving the readability of the syringe during this procedure.

2. <u>Description of the Prior Art</u>. Diabetic patients and those in need of taking other medication use a syringe to extract the proper dosage from a medication storage container and then deliver the medication to the body. For example, many diabetic patients maintain a glass vial or container holding a quantity of insulin sufficient for a number of doses. This vial has a needle-penetrable closure through which the syringe needle penetrates to extract the insulin. The patient must carefully insert the needle through the penetrable portion of the closure, withdraw the plunger of the syringe slowly while at the same time reading the scale imprinted on the syringe barrel to determine the amount of insulin being aspirated inside the syringe. Two problems are immediately apparent with this type of procedure.

It is appreciated that many of the individuals who must personally administer medication by way of needle delivery have infirmities which make it difficult to handle these instruments. Thus, in the case of the diabetic patient, the syringe needle is very small in diameter, as is the needle-penetrable portion of the closure on the insulin containing vial. Thus, these patients often find it difficult to initially insert this needle through the closure on the insulin containing vial. Furthermore, and particularly with low dosage requirements, the syringe itself is small and the volumetric numbers imprinted on the barrel of the syringe have small gradations. Patients with visual difficulties often have trouble in seeing these numbers and gradations accurately in order to assure that the proper dosage of the liquid medication is being withdrawn into the syringe from the vial.

With the foregoing problems in mind, it would be desirable to provide a device which would assist the user of a syringe-type instrument in guiding the syringe needle into the liquid medication container in order to withdraw the proper dosage. A steadying influence would contribute to making this proper insertion as well as minimizing the frustrations of the patient who experiences these kinds of difficulties. At the same time, it would also be desirable to magnify the imprinted numbers and gradations on the syringe to provide a visual amplification for those patients with visual difficulties. It is to satisfying these aims that the present invention is directed.

## SUMMARY OF THE INVENTION

The magnification guide of the present invention is useful in filling or emptying a liquid transfer instrument in conjunction with a liquid container. This guide comprises a transparent magnifying member having means for retaining a liquid container thereon during use. It also includes a guide means for guiding the instrument into liquid communication with the container during use. The magnifying member is adapted to magnify the surface of the instrument when positioned in the guide means.

In a preferred embodiment of the present invention, the magnification guide is useful in filling or emptying a syringe in conjunction with a liquid container having a narrow neck and a needle-penetrable closure thereon. The magnifying member is an elongate body having an open channel extending along the longitudinal axis of the body. This channel is adapted to receive the barrel of a syringe and guide the same toward the liquid container. An open recess is positioned near one end of the body extending deeper into the body than the channel. This recess is adapted to receive

425-9-1

3

the needle-penetrable closure of the liquid container to thereby retain the container in position on the body of the magnification guide. A transparent viewing surface is included on the body opposed from the channel which is adapted to magnify the appearance of the syringe barrel when positioned in the channel.

In accordance with the principles of the present invention, its structure provides significant improvements in at least two areas. One significant feature is the ability of this magnification guide to retain the liquid container (or vial) in a fixed position with respect to the guide. The long channel serves as an effective guide for the user who has trouble inserting the fine needle through the needle-penetrable closure. Thus, the present invention achieves the aim set forth above concerning an effective guide feature to assist the user particularly when loading the syringe. The second major feature of the present invention includes the magnifying surface. Once the user has guided the syringe along the guide channel, the transparent viewing surface of the invention magnifies the surface of the syringe barrel. With the volumetric numbers and gradations appearing larger to the user, it becomes significantly more convenient for the user to make sure that the proper dosage of liquid medication is in the syringe.

BRIEF DESCRIPTION OF THE DRAWINGS

Fig. 1 is a perspective view of the preferred magnification guide of the present invention useful in filling or emptying a syringe;

Fig. 2 is an end view of the magnification guide of Fig. 1 illustrating the end which includes a provision for attaching a liquid container thereto;

Fig. 3 is an enlarged plan view, in partial segment, of the end of the magnification guide of Figs. 1 and 2;

Fig. 4 is a perspective view of a liquid container of the type which is commonly employed for retaining liquid medications;

425-9-1

4

Fig. 5 is a perspective view of a syringe which includes small markings and gradations thereon;

Fig. 6 is a perspective view of the preferred magnification guide illustrating a liquid container, such as illustrated in Fig. 4, connected thereto;

Fig. 7 is a top, plan view of the combined structure of Fig. 6 further illustrating a syringe, such as shown in Fig. 5, being guided along the magnification guide and into the connected liquid container; and

Fig. 8 is a top, plan view of the opposite side of the magnification guide combination of Fig. 7, illustrating the viewing surface which magnifies the surface of the syringe barrel positioned on the guide.

## DETAILED DESCRIPTION

While this invention is satisfied by embodiments in many different forms, there is shown in the drawings and will herein be described in detail a preferred embodiment of the invention, with the understanding that the present disclosure is to be considered as exemplary of the principles of the invention and is not intended to limit the invention to the embodiment illustrated. The scope of the invention will be measured by the appended claims and their equivalents.

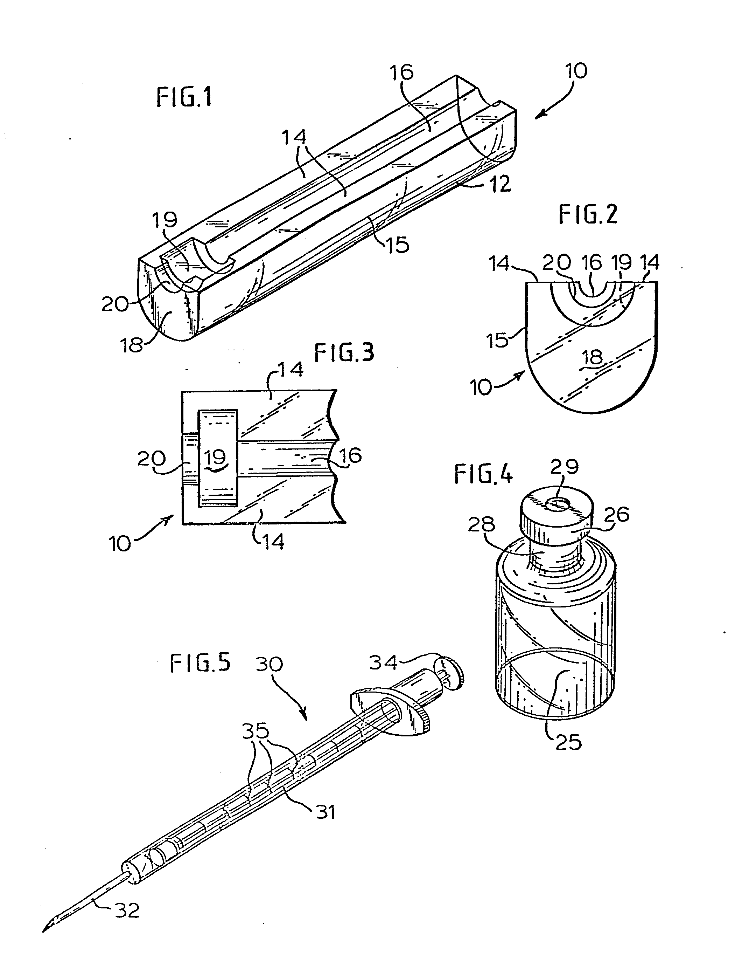

Adverting to the drawings, and Figs. 1-3 in particular, there is illustrated a magnification guide 10 in its preferred embodiment. Magnification guide 10 is preferably a unitary elongate body 12 made of transparent material, such as clear plastic. While body 12 may have many different configurations, the embodiment being described includes a flat surface 14 and a convex surface 15 spaced and opposed from the flat surface. This cross-sectional configuration has the appearance of a right-oval which has been cut in half, such as seen more clearly in Fig. 2. Depending into body 12 from flat surface 14 is an open, preferably semi-circular guide channel 16, which extends along the longitudinal axis of the body. Near end 18 of body 12 a recess 19 is formed, also depending into the body from flat surface 14. Recess 19 is also preferably

5

semi-circularly shaped and depends deeper into the body inasmuch as it has a radius greater than the radius of channel 16. This recess is spaced a short distance inwardly from end 18 by virtue of shoulder 20. This shoulder also is preferably semi-circularly shaped and has a radius intermediate the radii of channel 16 and recess 19. The respective lengths, along the longitudinal direction, of recess 19 and shoulder 20 are selected to be compatible with the respective lengths of closure 26 and neck portion 28 on the liquid container 25, such as illustrated in Fig. 4. Particularly for purposes of containing insulin, liquid container 25 generally has a standard configuration so that the length dimensions of closure 26 and neck 28 are consistent from container to container. Therefore, the lengths of recess 19 and shoulder 20, such as seen in Fig. 3, can be sized and spaced to receive the closure and the neck of the liquid container in a snap-fit connection. Concerning the dimensions of channel 16, it should have a radius compatible with the syringe 30, and in particular, its barrel 31 which will rest in the guide channel during use. The body of the magnification guide should be sufficiently long to provide guidance of the syringe into the liquid container, as described more fully hereinafter, but must not be so long that it interferes with the operation of the syringe, particularly during the filling or loading step.

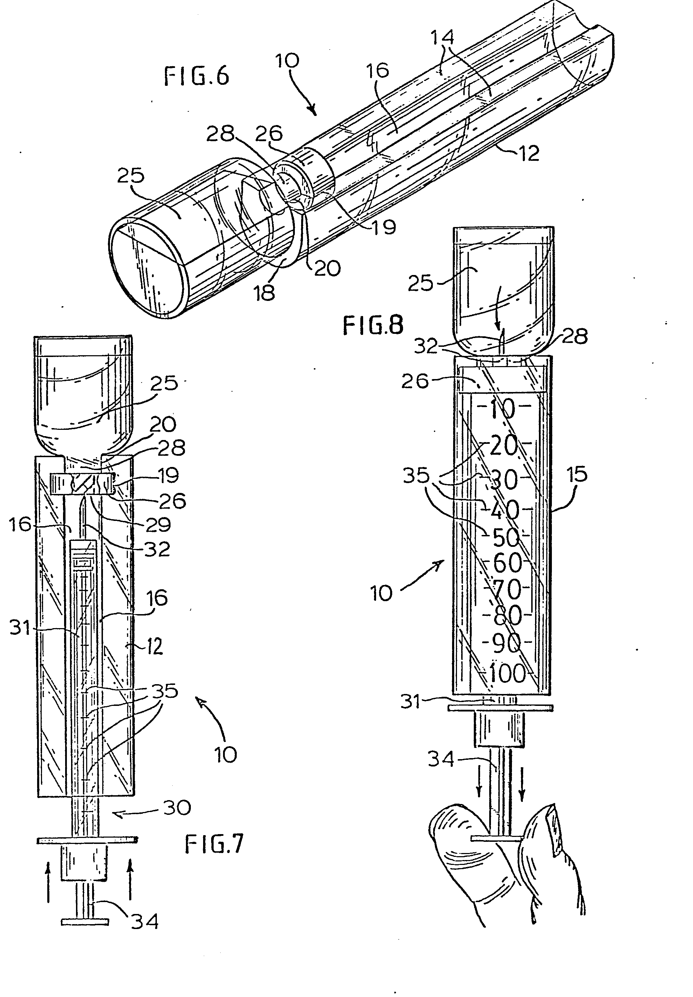

As seen in Fig. 5, syringe 30 is particularly useful in the delivery of insulin to a patient. This syringe also includes a fine hollow needle 32 connected to barrel 31. A slidable plunger 34 when withdrawn will allow liquid to be aspirated into the barrel. Volumetric gradations 35 are generally imprinted on the outside surface of the barrel, which itself is preferably transparent, so that the user can measure the amount of liquid flowing into the barrel and compare the level against one of the gradations for a determination of volumetric content. It is appreciated that, for low dosage applications, these gradations on a low dosage syringe are normally quite closely spaced with concomitant small

6

printed numbers. For individuals with visual difficulties, discerning the differences in these fine gradations can be troublesome when the liquid medication is being loaded into the syringe.

Turning now to Figs. 6, 7 and 8, the operation of the preferred magnification guide of the present invention is illustrated. Referring first to Fig. 6, liquid container 25 is shown attached to body 12 of the magnification guide. Closure 26 of the container is positioned into recess 19 of the magnification guide; neck portion 28 of the container rests on shoulder 20 of the magnification guide. This arrangement allows the container to be snap-fit onto the magnification guide so that a fixed connection is achieved between container and guide. This connection is, of course, only temporary while filling or emptying the syringe, since the liquid container can be removed by the user by applying a slight force to separate the snap-fit connection. In effect, magnification guide 10 becomes an extension or a handle for the user for holding the liquid container in a much more stable position in conjunction with the syringe. This operative step is illustrated in Fig. 7.

Syringe barrel 31 is positioned by the user into channel 16. The channel is sufficiently long so that the user can successfully accomplish this positioning essentially by feeling for the channel with the fingers and merely positioning the syringe therein. With a sliding movement, the user pushes the syringe until needle 32 penetrates through the penetrable portion 29 generally centrally located in closure 26 of the liquid container. The position of channel 16 with respect to the interconnected liquid container assures that sliding motion of the syringe will cause the needle to penetrate the closure so that the liquid medication in the container can be collected into the syringe. It can be seen that the hit-or-miss operation which users normally encounter is obviated by the structure of the present magnification guide. Once the syringe and needle are in proper position with respect to the liquid container, the user can start filling the syringe by withdrawing plunger 34, as more clearly seen by referring to Fig. 8.

In order to determine the amount of liquid medication withdrawn from container 25, the user need only turn magnification guide 10 around and look through clear convex surface 15. This curved surface serves to magnify the surface of syringe 30 which is positioned in channel 16. With this feature, gradations 35 appear much larger to the user as the syringe is being filled with liquid medication. This is especially advantageous for those individuals who have visual difficulties, but yet need to take this kind of medication themselves. Of course, when the syringe is filled or emptied, whichever the case may be, the user may separate container 25 from body 12 until such time as the next dosage is needed.

Thus, the present invention provides a magnification guide especially useful in filling or emptying syringe-type instruments. This magnification guide provides an improvement in inserting the syringe needle into a liquid container due to the guiding features which are structurally part of this invention. Furthermore, the magnification features allow the user to read the small gradations on the syringe more clearly so that the user can have a greater confidence in the accuracy of the dosage being prepared for delivery.

-8-

WHAT IS CLAIMED IS:

1. A magnification guide (10) for use in filling or emptying a syringe (30) in conjunction with a liquid container (25) having a narrow neck (28) and a needle-penetrable closure (26) thereon comprising:

an elongate body (12) having an open channel (16) extending along the longitudinal axis of said body (12), said channel (16) adapted to receive the barrel (31) of a syringe (30) and guide same toward the liquid container (25);

an open recess (19) near one end (18) of said body (12) extending deeper into said body (12) than said channel (16), said recess (19) adapted to receive the needle-penetrable closure (26) of the liquid container (25); and

said body (12) including a transparent viewing surface opposed from said channel (16) adapted to magnify the appearance of the barrel (31) when positioned in said channel (16).

2. The guide of claim 1 wherein said body (12) includes a flat surface (14) and a convex surface (15) spaced and opposed from said flat surface (14).

3. The guide of claim 2 wherein said channel (16) semi-circularly depends into said body (12) from said flat surface (14).

4. The guide of claim 3 wherein said recess (19) semi-circularly depends into said body (12) from said flat surface (14) on a greater radius than said channel (16), said recess (19) having a short length relative to the length of said channel (16).

5. The guide of claim 4 wherein said recess (19) is spaced a short distance inwardly from said end (18) and separated from said end by a semi-circularly shaped shoulder (20) depending into said body (12), said shoulder (20) having a radius intermediate the radii of said channel (16) and said recess (19).

6. The guide of claim 5 wherein said recess/(19) and said shoulder (20) are sized and spaced to receive the closure (26) and the neck (28) of the liquid container (25) in a snap-fit arrangement to thereby fixedly, but temporarily while filling or emptying said syringe (30), position said container (25) onto said body (12).

7. The guide of claim 1 wherein said body (12) is transparent plastic.

8. A magnification guide (10) for use in filling or emptying a liquid transfer instrument (30) in conjunction with a liquid container (25) comprising:
a transparent magnifying member (12) having means for retaining a liquid container (25) thereon during use and guide means (16,19) for guiding said instrument (30) into liquid communication with said container (25) during use, said member (12) adapted to magnify the surface of said instrument (30) when positioned in said guide means (16,19).

9. A magnification guide (10) for use in filling or emptying a syringe (30) in conjunction with a liquid container (25) having a narrow neck (28) and a needle-penetrable closure (26) thereon comprising:
an elongate transparent plastic body (12) including a flat surface (14) and a convex surface (15) spaced and opposed from said flat surface (14);

an open semi-circular guide channel (16) depending from said flat surface (14) into said body (12) extending along the longitudinal axis of said body (12), and adapted to receive the barrel (31) of a syringe (30) and guide same toward the liquid container (25); and

an open semi-circular recess (19) depending from said flat surface (14) into said body (12) near one end (18) thereof, said recess (19) having a greater radius than said channel (16) and adapted to receive the needle-penetrable closure (26) of the liquid container (25), said recess (19) being spaced a short distance inwardly from said end (18) and separated from said end (18) by a semi-circularly shaped shoulder (20) depending into said body (12), said shoulder (20) having a radius intermediate the radii of said channel (16) and said recess (19), said recess (19) and said shoulder (20) being sized and spaced to receive the closure (26) and the neck (28) of the liquid container (25) in a snap-fit arrangement to thereby fixedly, but temporarily while filling or emptying said syringe (30), position said container (25) onto said body (12), said convex surface (15) serving as a viewing surface through which the surface of said syringe barrel (31) is magnified when positioned in said channel (16).

1/2

FIG.1

FIG.2

FIG.3

FIG.4

FIG.5

FIG.6

10

28 26

25

16

14

12

19

20

18

FIG.8

25

32

28

26

35

15

-10-
-20-
-30-
-40-
-50-
-60-
-70-
-80-
-90-
-100-

10

31

34

25

20

28

19

26

29

32

16

16

31

12

35

10

30

34

FIG.7

European Patent
Office

**EUROPEAN SEARCH REPORT**

Application number

EP 82 10 0532

| | **DOCUMENTS CONSIDERED TO BE RELEVANT** | | |
|---|---|---|---|
| Category | Citation of document with indication, where appropriate, of relevant passages | Relevant to claim | CLASSIFICATION OF THE APPLICATION (Int. Cl. 3) |
| Y | US-A-3 610 241 (R.LEMARIE) *Column 1, lines 45-51; column 2, lines 4-6,22-48; figures 1,2* | 1-7,8 | A 61 J 1/00 |
| A | | 9 | |
| Y | US-A-1 726 314 (J.P.MARSCH AND CO.) *Page 1, lines 1-13; page 2, lines 15-19,41-68; figure 8* | 1-7,8 | |
| A | | 9 | |
| A | US-A-3 840 011 (F.A.WRIGHT) *Figures 1,3* | 6 | |
| A | EP-A-0 000 465 (A.BRISCHEWSKI) *Page 4, lines 2-13; page 6, lines 7-20; figure 5* | 6 | **TECHNICAL FIELDS SEARCHED (Int. Cl. 3)**<br><br>A 61 J<br>A 61 M<br>G 02 B |
| A | CH-A- 519 917 (O.NEUENSCHWANDER) | | |
| A | US-A-3 704 938 (H.FANSELOW) *Column 3, lines 21-31; figure 10* | 1-9 | |
| A | FR-A-1 602 925 (C.KAESER) | | |

The present search report has been drawn up for all claims

| Place of search | Date of completion of the search | Examiner |
|---|---|---|
| THE HAGUE | 17-09-1982 | ENGELBRECHT E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

&  : member of the same patent family, corresponding document

EPO Form 1503. 03.82